(19) Europäisches Patentamt / European Patent Office / Office européen des brevets

(11) **EP 2 950 082 B1**

(12) **EUROPEAN PATENT SPECIFICATION**

(45) Date of publication and mention
of the grant of the patent:
**15.09.2021 Bulletin 2021/37**

(51) Int Cl.:
*G01N 21/78* (2006.01)     *G01N 21/84* (2006.01)
*G01N 21/27* (2006.01)     *A61B 5/145* (2006.01)
*A61B 5/1455* (2006.01)    *A61B 5/1495* (2006.01)

(21) Application number: **14742983.1**

(22) Date of filing: **20.01.2014**

(86) International application number:
**PCT/JP2014/050905**

(87) International publication number:
**WO 2014/115666 (31.07.2014 Gazette 2014/31)**

(54) **CALIBRATION METHOD, CALIBRATION SYSTEM, AND BODILY-FLUID COMPONENT MEASUREMENT DEVICE CALIBRATED USING SAID METHOD**

KALIBRIERUNGSVERFAHREN, KALIBRIERUNGSSYSTEM UND IN DIESEM VERFAHREN KALIBRIERTE VORRICHTUNG ZUR MESSUNG VON KÖRPERFLÜSSIGKEITSKOMPONENTEN

PROCÉDÉ D'ÉTALONNAGE, SYSTÈME D'ÉTALONNAGE ET DISPOSITIF DE MESURE DE COMPOSANT DE FLUIDE CORPOREL ÉTALONNÉ AU MOYEN DUDIT PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.01.2013 JP 2013010616**

(43) Date of publication of application:
**02.12.2015 Bulletin 2015/49**

(73) Proprietor: **Terumo Kabushiki Kaisha
Tokyo 151-0072 (JP)**

(72) Inventors:
• **AIKAWA, Ryokei
Ashigarakami-gun, Kanagawa 259-0151 (JP)**
• **MOMOKI, Hideyuki
Ashigarakami-gun, Kanagawa 259-0151 (JP)**

(74) Representative: **Casalonga
Casalonga & Partners
Bayerstraße 71/73
80335 München (DE)**

(56) References cited:
**WO-A1-2009/123069     WO-A1-2012/086376
WO-A1-2012/128014     WO-A1-2012/128014
WO-A1-2012/147504     JP-A- H11 183 377
JP-A- 2005 151 036      JP-A- 2005 214 806
JP-A- 2005 214 806      JP-A- 2010 230 663
JP-A- 2012 198 083      US-A1- 2005 286 053**

• **None**

## Description

Technical Field

The present invention relates to a calibration method and a calibration system for calibrating a body fluid component measuring instrument configured to include a measurement optical system for projecting light on a measurement surface of a test strip that contains a chromogenic reagent that reacts with a component in a body fluid and detecting reflected light, and configured to measure the component in the body fluid based on a detection light quantity of the reflected light obtained by the measurement optical system, and also relates to a body fluid component measuring instrument calibrated by using the same method.

Background Art

WO2012/147504 relates to a colorimeter for measuring metallic painting for a car or the like. WO2012/128014 relates to a component measuring device.
There has been known a blood glucose meter capable of measuring a blood glucose level of a subject as an embodiment of a body fluid component measuring instrument for measuring a component in a body fluid. For example, an optical blood glucose meter is configured to supply blood to a test strip impregnated with a reagent that develops color corresponding to the amount of glucose in the blood and to optically detect a property of the test strip so as to calculate the blood glucose level.
JP 9-145615 A discloses an instrument that has an optical system (hereinafter, referred to as a measurement optical system) that includes a light source for projecting light on a measurement surface of the test strip and a sensor for detecting the reflected light.

Summary of Invention

In manufacturing the instrument disclosed in the above-described JP 9-145615 A, if mounting accuracy of the measurement optical system is low, it may not be possible to obtain desired measurement accuracy due to optical axis deviation that may occur in the measurement optical system.
In order to manage mounting accuracy of the measurement optical system more precisely, however, more time and processes for a mounting operation would be needed. As a result, there has been a problem of increased manufacturing costs.
The present invention is intended to solve the above-described problem. The object of the present invention is to provide a calibration method and a calibration system capable of ensuring accuracy of measuring a component in a body fluid without precisely managing mounting accuracy of the measurement optical system, and to provide a body fluid component measuring instrument calibrated by using the same method.
The calibration method according to the present invention is a method for calibrating a body fluid component measuring instrument configured to include a measurement optical system for projecting light on a measurement surface of a test strip that contains a chromogenic reagent that reacts with a component in a body fluid and detecting reflected light, and configured to measure the component in the body fluid based on a detection light quantity of the reflected light obtained by the measurement optical system. The calibration method includes: a measurement step of measuring an offset of a reflection point on the measurement surface, the offset representing a deviation amount of an actual optical path of the measurement optical system with respect to a reference optical path of the measurement optical system, the measurement optical system being mounted on a calibration target instrument that is the body fluid component measuring instrument as a calibration target; and a determination step of calculating a correction light quantity to be uniformly added or subtracted to/from the detection light quantity based on the measured offset and determining a calibration curve for the calibration target instrument based on the correction light quantity.
The reflected light detected by the measurement optical system not only includes scattered light that has been scattered inside the test strip and emitted to the outside, but also includes surface reflection light reflected as it is on the measurement surface of the test strip. The surface reflection light has an angle distribution such that light intensity is maximized at a specular reflection angle, and tends to have a uniform light intensity regardless of the amount of component in the body fluid. That is, a changing optical path (namely, reflection angle) of the measurement optical system leads to a different detection light quantity attributed to the surface reflection light.
Accordingly, an offset of the reflection point on the measurement surface of the test strip is measured, the offset representing a deviation amount of the actual optical path of the measurement optical system with respect to the reference optical path. Based on the offset, a correction light quantity to be uniformly added or subtracted to/from the detection light quantity is calculated. Based on the correction light quantity, a calibration curve for the calibration target instrument is determined. Consequently, it is possible to execute calibration that cancels a change in the detection light quantity attributed to the surface reflection light. As a result, it is possible to ensure accuracy of measuring a component in a body fluid without precisely managing mounting accuracy of the measurement optical system.

Furthermore, the measurement step preferably measures, as the offset, deviation of the reflection point along an intersection line formed by a surface that contains the reference optical path and the measurement surface. According to geometrical considerations, since the rate of change in a reflection light quantity is maximized when the optical path (reflection point) varies along the intersection line, the effect of this calibration becomes remarkable.

The body fluid component measuring instrument preferably measures the component in the body fluid based on a ratio of the detection light quantities on the test strip between before and after color development. The determination step preferably determines the calibration curve based on a correction model configured to add or subtract the correction light quantity to/from each of the detection light quantities, respectively, before and after color development. This can suppress deterioration of measurement accuracy when a degree of contribution of the surface reflection light is relatively large, that is, when a ratio of the detection light quantity is small.

Furthermore, the determination step preferably acquires association information that represents a relationship of the correction light quantity with respect to the offset and preferably calculates the correction light quantity with reference to the association information. This makes it possible to easily execute calculation of the correction light quantity and determination of the calibration curve, leading to improved operation efficiency.

The calibration system according to the present invention is configured to calibrate a body fluid component measuring instrument configured to include a measurement optical system for projecting light on a measurement surface of a test strip that contains a chromogenic reagent that reacts with a component in a body fluid and detecting reflected light, and configured to measure the component in the body fluid based on a detection light quantity of the reflected light obtained by the measurement optical system. The calibration system includes: offset measurement means configured to measure an offset of a reflection point on the measurement surface, the offset representing a deviation amount of an actual optical path of the measurement optical system with respect to a reference optical path of the measurement optical system, the measurement optical system being mounted on a calibration target instrument that is the body fluid component measuring instrument as a calibration target; and calibration curve determination means configured to calculate a correction light quantity to be uniformly added or subtracted to/from the detection light quantity based on the offset measured by the offset measurement means and configured to determine a calibration curve for the calibration target instrument based on the correction light quantity.

The body fluid component measuring instrument according to the present invention is an instrument calibrated by using any of the above-described calibration methods. The body fluid component measuring instrument preferably includes a storage unit configured to store a calibration coefficient that specifies the determined calibration curve, and preferably includes a component quantification section configured to use the calibration coefficient read from the storage unit to uniformly add or subtract the correction light quantity to/from the detection light quantity obtained by the measurement optical system and then to quantify the component in the body fluid.

With the calibration method, calibration system, and the body fluid component measuring instrument calibrated by using the same method, according to the present invention, an offset of the reflection point on the measurement surface of the test strip is measured, the offset representing a deviation amount of the actual optical path of the measurement optical system with respect to the reference optical path. Based on the offset, a correction light quantity to be uniformly added or subtracted to/from the detection light quantity is calculated. Based on the correction light quantity, a calibration curve for the calibration target instrument is determined.

The reflected light detected by the measurement optical system not only includes scattered light that has been scattered inside the test strip and emitted to the outside, but also includes surface reflection light reflected as it is on the measurement surface of the test strip. The surface reflection light has an angle distribution such that light intensity is maximized at a specular reflection angle, and tends to have a uniform light intensity regardless of the amount of component in the body fluid. That is, a changing optical path (namely, reflection angle) of the measurement optical system leads to a different detection light quantity attributed to the surface reflection light.

Accordingly, the above configuration, if adopted, can execute calibration that cancels a change in the detection light quantity attributed to the surface reflection light. As a result, it is possible to ensure accuracy of measuring a component in a body fluid without precisely managing mounting accuracy of the measurement optical system.

Brief Description of Drawings

FIG. 1 is a perspective view of a blood glucose meter as a body fluid component measuring instrument of the present embodiment.
FIG. 2 is a schematic block diagram of the blood glucose meter illustrated in FIG. 1.
FIG. 3 is an electrical block diagram of a calibration system for calibrating the blood glucose meter illustrated in FIG. 1.

FIG. 4 is a flowchart illustrating an operation of the calibration system illustrated in FIG. 3.

FIG. 5 is a schematic diagram of a definition method for plane coordinates on a measurement surface of the test strip.

FIG. 6 is an exemplary diagram of an image obtained by imaging the periphery of the measurement optical system illustrated in FIGS. 2 and 3.

FIGS 7A and 7B are schematic diagrams illustrating angle distributions of surface reflection light to be generated on the measurement surface of the test strip.

FIG. 8 is a graph illustrating an exemplary relationship of a degree of change in the light quantity with respect to an offset.

FIG. 9 is a graph illustrating an exemplary quantification curve.

Description of Embodiments

Hereinafter, a calibration method, a calibration system, and a body fluid component measuring instrument calibrated by using the same method, according to the present invention, will now be described with preferred embodiments and attached drawings.

[Configuration of Blood Glucose Meter 10]

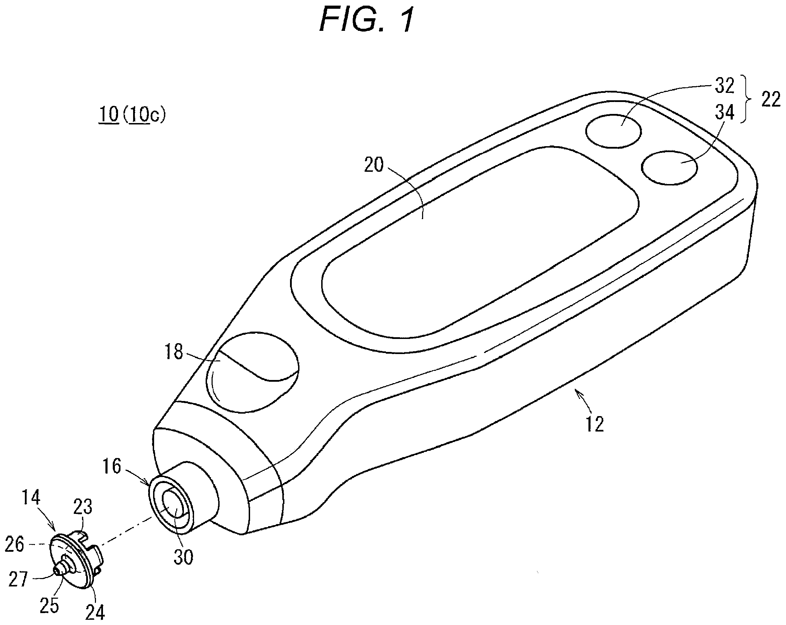

FIG. 1 is a perspective view of a blood glucose meter 10 as a body fluid component measuring instrument of the present embodiment.

The blood glucose meter 10 includes a body 12, a tip attachment section 16 to which a tip 14 is attached, an ejector 18 provided on an upper surface near the tip attachment section 16, a liquid crystal panel 20 provided at a center of an upper surface of the body 12, and an operation section 22 provided at a proximal end on the upper surface of the body 12.

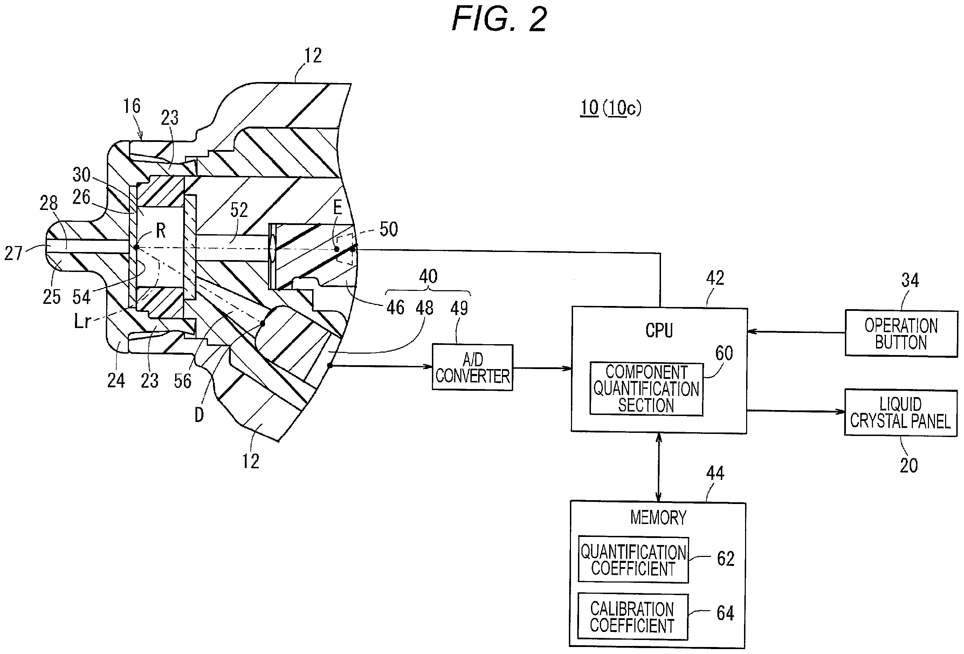

At a leading end of the blood glucose meter 10, the tip 14 as a test device is attached. The tip 14 includes a base cylinder 23 of a bottomed-cylindrical shape, a flange 24 protruding from the base cylinder 23 in a radially outward direction, a nozzle 25 protruding from a base section of the base cylinder 23, and a test strip 26 provided at an inner bottom surface of the base cylinder 23. At a center of the nozzle 25, a linear blood guide passage 28 (FIG. 2) is provided, passing from a spotting section 27 at the leading end through the test strip 26.

As a material of the test strip 26, polyether sulfone is used, for example. Examples of chromogenic reagents to be impregnated on the test strip 26 include color developers such as glucose oxidase (GOD), peroxidase (POD), 4-aminoantipyrine, N-ethyl-N-(2-hydroxy-3-sulfopropyl). The reagent may include a predetermined buffer agent.

The body 12 of the blood glucose meter 10 has an elongated shape easily gripped with one hand by a user. A leading end section of the body tapers toward a leading end and is bent slightly in a lower direction, making it possible to perform a blood spotting operation easily.

The tip attachment section 16 is provided at the leading end section of the blood glucose meter 10 and is configured to be cylindrical such that the above-described tip 14 can be attached thereto. When a user measures a blood glucose level by using the blood glucose meter 10, the tip 14 is attached to the tip attachment section 16, and then, blood is drawn from the spotting section 27 on the leading end of the attached tip 14. At the center of the tip attachment section 16, a measurement window 30 is provided. At a time of a measurement, light projection and light reception are performed by a measurement optical system 40 (FIG. 2) via the measurement window 30.

The ejector 18, in response to a pressing operation in a forward direction, pushes out the attached tip 14 in the forward direction to detach the tip from the tip attachment section 16. As a result, it is possible to discard the tip 14 on which a blood glucose level measurement has been completed.

The liquid crystal panel 20 displays a measured blood glucose level as a measurement result, and can display an operation procedure guide, therapeutic guidance, and information on an error that has been encountered, together with countermeasures for the error, or the like. The operation section 22 has a power button 32 (not illustrated) for turning on/off the power source, and an operation button 34 for performing operations such as setting and inputting of various types of data.

[Electrical Block Diagram of Blood Glucose Meter 10]

FIG. 2 is a schematic block diagram of the blood glucose meter 10 illustrated in FIG. 1. Note that in the left portion of the diagram, a partly enlarged sectional view of the leading end of the blood glucose meter 10 is illustrated.

The blood glucose meter 10 includes, in addition to the liquid crystal panel 20 and the operation button 34, the measurement optical system 40, a central processing unit (hereinafter, referred to as a CPU 42), and a memory 44 (storage unit).

The measurement optical system 40 includes a light projecting section 46 configured to illuminate the test strip 26 with pulsed light, a light receiving section 48 configured to receive reflected light from the test strip 26, and an A/D converter 49 configured to convert an analog signal obtained by photoelectric conversion at the light receiving section 48 into a digital signal.

At the light projecting section 46, a light emitting device 50 that emits light is disposed. Examples of various types of devices that can be used as the light emitting device 50 include: a light emitting diode (LED), an organic electroluminescence (EL) device, an inorganic EL device, and a laser diode (LD) device.

The light receiving section 48 is formed of a light receiving device that converts detected light into an electrical signal. Examples of various types of devices that can be used as the light receiving device include a photodiode (PD) device, a charge coupled device (CCD), and a complementary metal oxide semiconductor (CMOS) device.

The light emitted from the light emitting device 50 passes straight through inside a light projecting path 52 to be reflected on a measurement surface 54 of the test strip 26, passing straight through a light receiving path 56 to be received by the light receiving section 48.

The measurement optical system 40 has been optically adjusted such that a light projecting region on the measurement surface 54 is substantially a circular shape with a diameter of 1 to 3 mm. Hereinafter, the V-shaped optical path that connects three points, that is, a light emission point E that specifies the position of the light projecting section 46, a reflection point R on the measurement surface 54, and a light receiving point D that specifies the position of the light receiving section 48, is referred to as an "actual optical path Lr".

The CPU 42, by reading and executing a program stored in the memory 44, or the like, performs an operation for controlling each of components including the measurement optical system 40 and, furthermore, enables functions of a component quantification section 60 that quantifies the blood glucose level based on a signal value output from the A/D converter 49.

The memory 44 is a volatile or non-volatile storage medium. According to the exemplary diagram, a quantification coefficient 62 and a calibration coefficient 64 are each stored in the memory 44. Here, the quantification coefficient 62 is a coefficient for specifying a quantification curve, and the calibration coefficient 64 is a coefficient for specifying a calibration curve.

[Electrical Block Diagram of Calibration System 70]

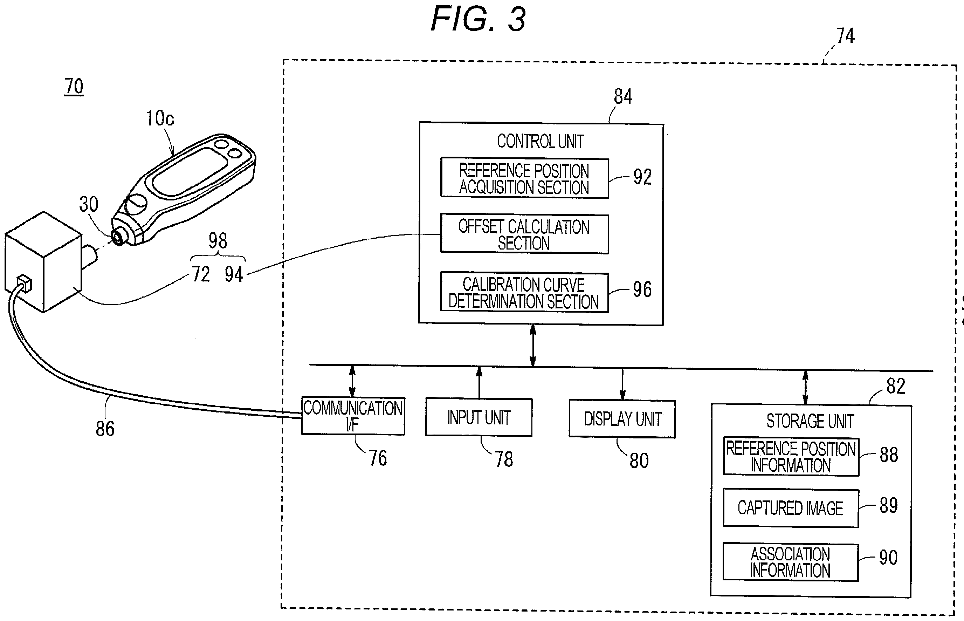

FIG. 3 is an electrical block diagram of a calibration system 70 that calibrates the blood glucose meter 10 illustrated in FIG. 1. The calibration system 70 basically includes an imaging apparatus 72 configured to capture an image of an inside of the blood glucose meter as a calibration target (hereinafter, also referred to a calibration target instrument 10c), and a calibration operation terminal 74 configured to calibrate the calibration target instrument 10c based on an image signal that has been output from the imaging apparatus 72.

The imaging apparatus 72 has an image sensor (imaging device array) that converts an input light image into an electrical signal. Examples of various types of devices that can be used as the imaging device include a PD device, a CCD, and a CMOS device. The image signal (captured image 89) output from the imaging apparatus 72 may be a monochrome image or a color image.

The calibration operation terminal 74 is a computer equipped with a communication I/F 76, an input unit 78, a display unit 80, a storage unit 82 and a control unit 84.

The communication I/F 76 is an interface for transmitting/receiving an electrical signal to/from an external device (the imaging apparatus 72 in an example in FIG. 3). The imaging apparatus 72 is communicably connected with the calibration operation terminal 74 via a cable 86. The imaging apparatus 72 and the calibration operation terminal 74 may be communicably connected wirelessly instead of using wire communication via the cable 86.

The input unit 78 is configured with various input devices including a mouse, a track ball, and a keyboard. The display unit 80 is a unit capable of monochrome or color display and may be configured with a liquid crystal panel, an organic EL panel, an inorganic EL panel, or the like.

The storage unit 82 stores a program, data, or the like, needed for the control unit 84 to control each component. According to the exemplary diagram, reference position information 88, the captured image 89, and association information 90 are each stored in the storage unit 82. Note that the storage unit 82 may be a nontransitory and computer readable storage medium including a non-volatile memory, and a hard disk.

The control unit 84 is configured with a processor such as a central processing unit (CPU). The control unit 84, by reading and executing a program stored in the storage unit 82, enables each of functions of a reference position acquisition section 92, an offset calculation section 94, and a calibration curve determination section 96 (calibration curve determination means).

Combining an imaging function achieved by the imaging apparatus 72 with a calculation function achieved by the offset calculation section 94 makes it possible to function as offset measurement means 98 for measuring an offset ($\Delta$X, $\Delta$Y) to be described later.

[Operation of Calibration System 70]

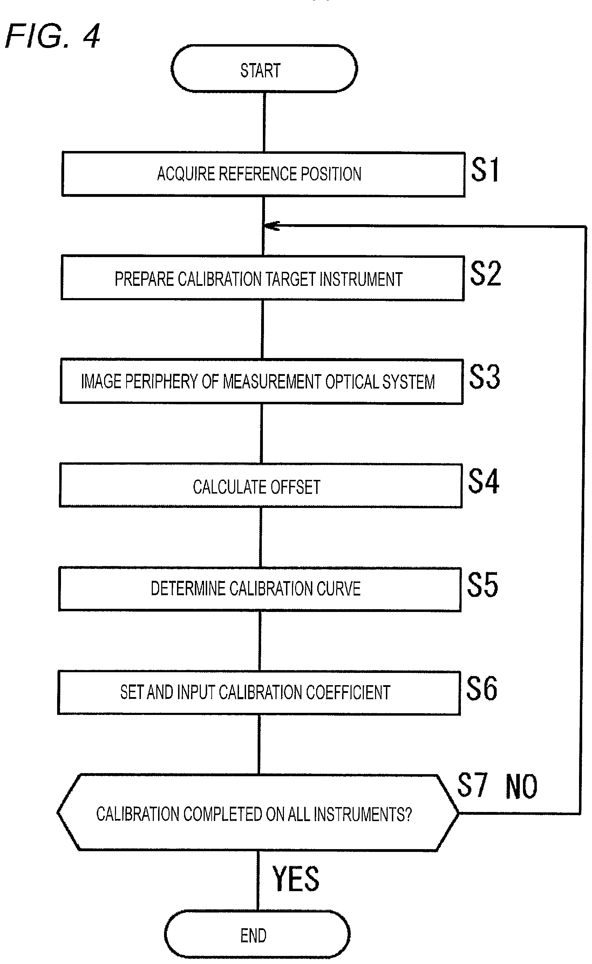

Next, an operation of the calibration system 70 illustrated in FIG. 3 will be described with reference to a flowchart illustrated in FIG. 4.

At step S1, the reference position acquisition section 92 reads the preliminarily stored reference position information 88 to acquire a reference position for specifying a reference optical path Ls of the measurement optical system 40.

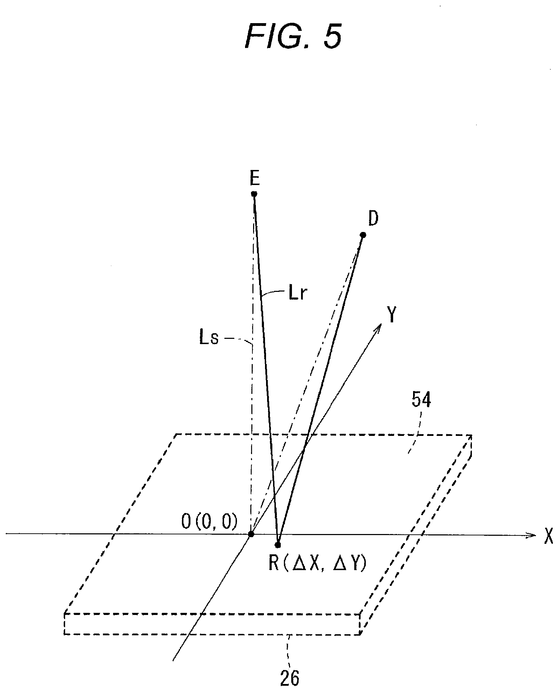

FIG. 5 is a schematic diagram of a definition method for plane coordinates on the measurement surface 54 of the test strip 26. The V-shaped dotted chain line represents a reference optical path (reference optical path Ls) on the measurement optical system 40. The reference optical path Ls corresponds to, for example, a target optical path of the measurement optical system 40. In this case, if a vertex of the V-shape is defined as "reference reflection point O", the line segment OE becomes a normal of the measurement surface 54. Then, a direction along an intersection line formed by a surface containing the reference optical path Ls and the measurement surface 54 is defined as an X-axis, and a direction orthogonal to both of the X-axis and the line segment OE is defined as a Y-axis.

At step S2, a calibration operator prepares the blood glucose meter 10 as a calibration target, namely, the calibration target instrument 10c. At this time, the tip 14 has not been attached to the tip attachment section 16 of the calibration target instrument 10c.

At step S3, the imaging apparatus 72 is used to image the periphery of the measurement optical system 40 already mounted on the calibration target instrument 10c. For example, under the state in which a screen for imaging is disposed as a substitute for the test strip 26, turn-on control is executed on the light emitting device 50. Then, the calibration operator turns an imaging surface of the imaging apparatus 72 to the measurement window 30 and captures an image of the light projected on the screen from the light emitting device 50. The image signal that has been output from the imaging apparatus 72 is supplied to the calibration operation terminal 74 via the cable 86 and the communication I/F 76. Thereafter, the control unit 84 temporarily stores the image signal in the storage unit 82 as the captured image 89.

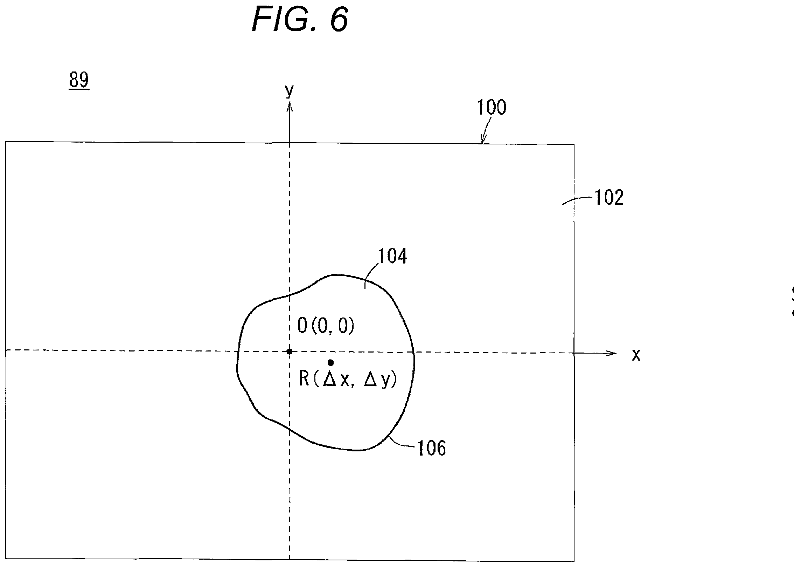

FIG. 6 is an exemplary diagram of an image obtained by imaging the periphery of the measurement optical system 40 illustrated in FIGS. 2 and 3. More particularly, the diagram corresponds to the diagram of the captured image 89 that has been schematically visualized.

An image region 100 of the captured image 89 includes a background region 102 having a relatively low luminance region, and a bright region 104 having a relatively high luminance region. The bright region 104 corresponds to a projected light image from the light projecting section 46 (FIG. 2). For convenience of illustration, the boundary line between the background region 102 and the bright region 104 is solely described in the image region 100.

At step S4, the offset calculation section 94 calculates the offset of the reflection point R on the measurement surface 54 based on the captured image 89 obtained at step S3. Hereinafter, definition of the offset will be described referring back to FIGS. 5 and 6.

Referring back to FIG. 5, the V-shaped solid line represents an actual optical path (actual optical path Lr: refer to FIG. 2) of the measurement optical system 40 mounted on the calibration target instrument 10c. As understandable from the diagram, the actual optical path Lr deviates in the positive direction on the X-axis and in the negative direction on the Y-axis, with respect to the reference optical path Ls. Here, a deviation amount of the actual optical path Lr with respect to the reference optical path Ls is quantified as the offset $(\Delta X, \Delta Y)$ of the reflection point R on the measurement surface 54 of the test strip 26. The reflection point R corresponds to a vertex of the V-shaped actual optical path Lr.

According to the exemplary diagram, the light emission points E, one for the reference optical path Ls and the other for the actual optical path Lr, coincide with each other. In this case, deviation only exists in mounting orientation of the light projecting section 46. Together with this, or apart from this, there is a case where the mounting position of the light projecting section 46 deviates. At that time, the light emission point of the actual optical path Lr does not coincide with the light emission point E of the reference optical path Ls.

Referring back to FIG. 6, the offset calculation section 94 uses a known image recognition processing to analyze the captured image 89, thereby specifying plane coordinates of the X and Y axes (including the position of the reference reflection point O) and calculating coordinates of the reflection point R. For example, the plane coordinates of the X and Y axes may be specified by recognizing the shape or pattern position/orientation of each member captured on the captured image 89 (particularly in the background region 102). Furthermore, the coordinates of the reflection point R $(\Delta X, \Delta Y)$ may be calculated by calculating a gravity center position $(\Delta x, \Delta y)$ of the bright region 104 as the reflection point R, and then converting the value to a length in real space by using various information (including image resolution and optical magnification) on the imaging apparatus 72.

Hereinafter, deviation $\Delta X$ of the reflection point R along the X-axis and deviation $\Delta Y$ of the reflection point R along the Y-axis are, in some cases, referred to "X-axis offset" and "Y-axis offset", respectively. In the present embodiment, the X-axis offset $\Delta X$ alone is used among amounts of vectors $(\Delta X, \Delta Y)$. The reason will be described later.

At step S5, the calibration curve determination section 96 determines the calibration coefficient 64 suitable for the calibration target instrument 10c, based on the offset obtained in step S4. Before description of a specific determination method is given, an optical phenomenon related to mounting accuracy of the measurement optical system 40 will be described.

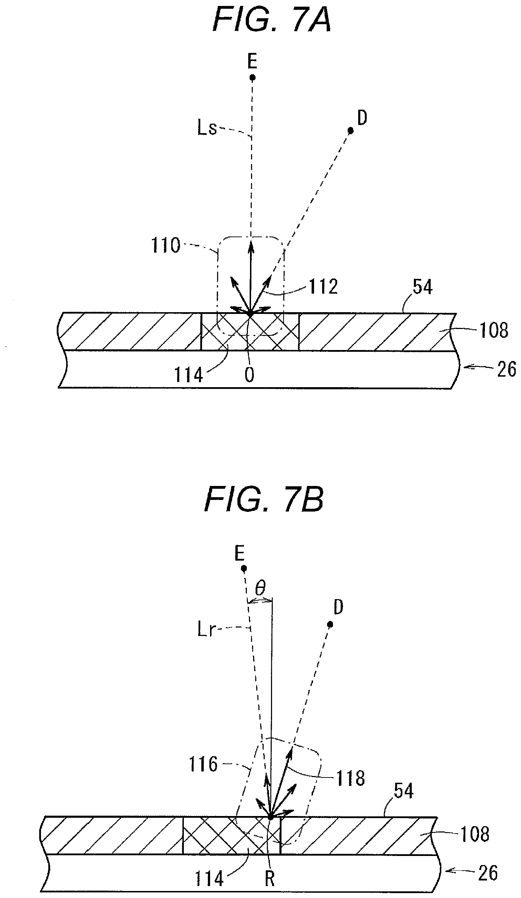

FIGS 7A and 7B are schematic diagrams illustrating angle distributions of surface reflection light 110 and 116 to be generated on the measurement surface 54 of the test strip 26.

As illustrated in FIG. 7A, irradiated light is assumed to go along the reference optical path Ls (incident angle: zero) that starts from the light emission point E and ends on the light receiving point D, and assumed to be detected. The detected light not only includes scattered light that has been scattered inside the test strip 26 (layer 108 impregnated with chromogenic reagent) and is emitted to the outside, but also includes the surface reflection light 110 that has been reflected as it is on the measurement surface 54 of the test strip 26.

The length of each of the arrows of the surface reflection light 110 indicates the level of light intensity. The longer the arrow is, the higher the light intensity is. The shorter the arrow is, the lower the light intensity is. That is, the surface reflection light 110 has an angle distribution in which light intensity is maximized at a specular reflection angle (reflection angle: zero). Consequently, as illustrated in the exemplary figure, a reflection component 112 that has the second highest light intensity is received and detected.

On the other hand, as illustrated in FIG. 7B, irradiated light is assumed to go along the actual optical path Lr (incident angle: $\theta$) that starts from the light emission point E and ends on the light receiving point D, and then to be detected. Similarly to the case in FIG. 7A, detected light includes the surface reflection light 116 that has been reflected as it is on the measurement surface 54 of the test strip 26. The surface reflection light 116 has an angle distribution in which light intensity is maximized at a specular reflection angle (reflection angle: $\theta$). Accordingly, a reflection component 118 that has the highest light intensity is received and detected.

In this manner, a changing an optical path of the measurement optical system 40, namely, the reflection angle leads to a difference in the detection light quantity attributed to the surface reflection light 110 and 116. In addition, this phenomenon is mainly attributed to surface reflection, substantially the same tendency is found regardless of the amount of component in a body fluid (color of a color developing section 114). Thus, the calibration method according to the present embodiment determines the calibration coefficient 64 that cancels the above-described change in the detection light quantity.

At this determination, the calibration curve determination section 96 reads from the storage unit 82 the association information 90 that represents a relation of a correction light quantity ($\Delta I$) to an offset and acquires the information.

In particular, it is preferable that deviation ($\Delta X$) of the reflection point R along the intersection line (X-axis) formed by a surface that contains the reference optical path Ls and the measurement surface 54 is measured and used as an offset. According to geometrical considerations, the rate of change in a reflection light quantity is maximized when the optical path (reflection point R) varies along the X-axis, the effect of this calibration becomes remarkable.

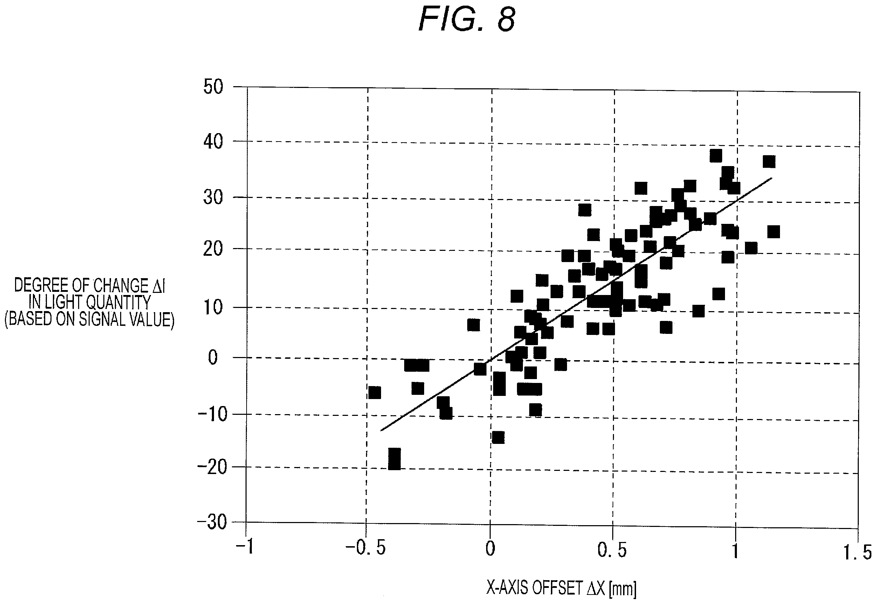

FIG. 8 is a graph illustrating an exemplary relationship of a degree of change $\Delta I$ in the light quantity with respect to an offset. In the graph, the horizontal axis represents an X-axis offset $\Delta X$ (unit: mm), and the vertical axis represents a degree of change $\Delta I$ in the light quantity (unit: none). Here, $\Delta I$ corresponds to the quantity that has been converted to the signal value output from the A/D converter 49 (FIG. 2).

The figure plots data points of experimental data obtained by varying mounting position/orientation of the measurement optical system 40. The solid line in the figure represents a regression line determined from the individual plots. When the intercept is zero, the function is represented as: $\Delta I (\Delta X) = C1 \cdot \Delta X$ (C1: positive number).

Note that this relational expression is not limited to the line having an intercept of zero. Any linear function or nonlinear function can be used. As an optimization technique for fitting into a predetermined curve, various known techniques including function approximation operation, fitting operation, interpolation operation and multivariate analysis, are applicable.

Furthermore, the association information 90 may also be a lookup table (LUT), in addition to be a coefficient for specifying the function shape. Furthermore, the association information 90 may include not only the relation with the X-axis offset $\Delta X$ but also the relation with the Y-axis offset $\Delta Y$, together with, or separately from the X-axis offset $\Delta X$.

The calibration curve determination section 96, by referencing the association information 90, obtains $\Delta I$ that corresponds to $\Delta X$ that has been obtained in step S4. This makes it possible to easily execute determination of the correction light quantity ($\Delta I$) and calculation of the calibration curve (calibration coefficient 64), leading to improved operation efficiency. The calibration curve determination section 96 determines ($-\Delta I$) (regardless of presence/absence of sign) for canceling the degree of change as the calibration coefficient 64.

At step S6, the calibration operator sets and inputs the calibration coefficient 64 (FIG. 2) determined at step S5 into the calibration target instrument 10c. For example, the calibration coefficient 64 may be input via the operation button 34 (FIG. 1) or may be downloaded from the calibration operation terminal 74 (FIG. 3) through a communication method (not illustrated). Accordingly, the calibration coefficient 64 suitable for each of the blood glucose meters 10 is each stored in the memory 44.

At step S7, the calibration operator confirms whether calibration operations on all calibration target instruments 10c are completed. If not completed, the procedure goes back to step S2, and then, steps S2 to S6 are repeated sequentially. If completed, the calibration operations for the blood glucose meter 10 are finished. In this manner, the user can use the blood glucose meter 10 in a calibrated state.

[Quantitative Equation of Blood Glucose Level by Blood Glucose Meter 10]

Next, a quantification method for the blood glucose level by using the calibrated blood glucose meter 10, and more particularly, a computing operation at the component quantification section 60 (FIG. 2) will be described.

The component quantification section 60, prior to computing, reads the quantification coefficient 62 and the calibration coefficient 64 stored in the memory 44, and also acquires two values of the detection light quantities that have been output via the measurement optical system 40. The detection light quantity on the test strip 26 before color development is defined as Iw, and the detection light quantity on the test strip 26 after color development is defined as Ic. Normally, a ratio of the detection light quantities (Ic/Iw) on the test strip 26 between before and after color development is calculated as optical reflectance. In the present embodiment, however, calibration of the blood glucose meter 10 is executed by calculating optical reflectance $\eta$ that cancels a change in the detection light quantity attributed to the surface reflection light 110 and 116 (FIGS. 7A and 7B).

The calibrated optical reflectance $\eta$ is calculated based on a correction model represented by the following equation (1).

$$\eta = (Ic - \Delta I)/(Iw - \Delta I) \ ... \ (1)$$

Here, $\Delta I$ is a degree of change in the light quantity (correction light quantity) illustrated in FIG. 8 and corresponds to the calibration coefficient 64 individually determined for each of the blood glucose meters 10. That is, when the X-axis offset $\Delta X$ is a positive value, subtraction is executed for each of detection light quantities Ic and Iw. On the other hand, when the X-axis offset AX is a negative value, addition is executed for each of the detection light quantities Ic and Iw.

Absorbance Ab is calculated using the optical reflectance $\eta$ based on the following equation (2).

$$Ab = 1024/\eta \ ... \ (2)$$

Here, the optical reflectance $\eta$ is within the range of $0 \le \eta \le 1$, therefore, the absorbance Ab is understandably a positive value, the minimum value thereof being 1024.

The blood glucose level BG (unit: mg/dl) is calculated using the absorbance Ab and any function F ($\cdot$), based on the following equation (3).

$$BG = F(Ab) \ ... \ (3)$$

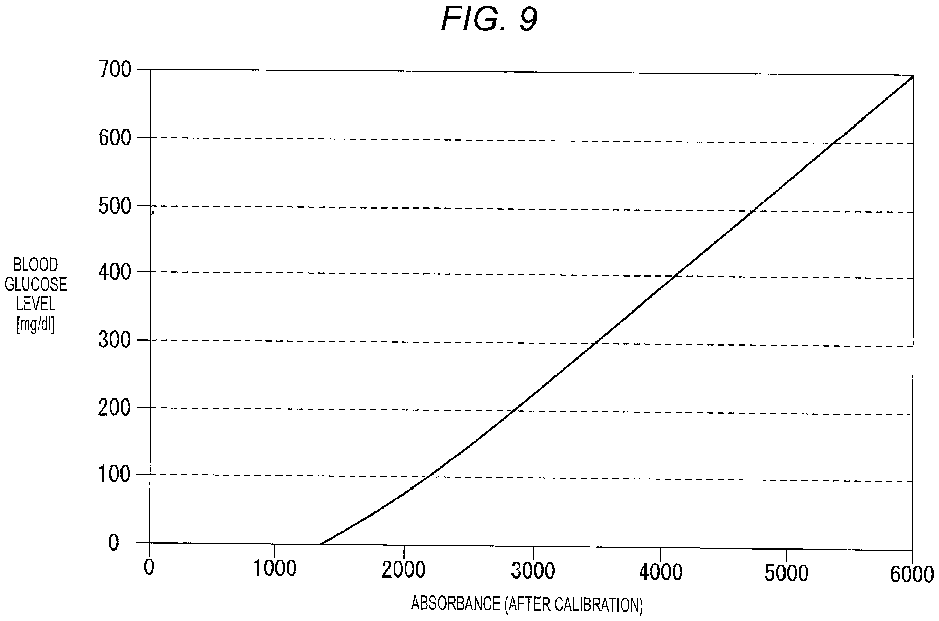

FIG. 9 is a graph illustrating an exemplary quantification curve. In the graph, the horizontal axis represents the absorbance Ab (unit: none) and the horizontal axis represents a blood glucose level BG (unit: mg/dl). In the graph, the blood glucose level BG is expressed by a cubic equation of the absorbance Ab. In this case, the quantification coefficient 62 includes four coefficients for uniquely determining the shape of the function.

Note that methods for calculating the blood glucose level are not limited to equations (1) to (3). Various computation methods based on the optical reflectance $\eta$ are understandably applicable. In the correction model represented by (1), the correction light quantity ($\Delta I$) is added or subtracted to/from the detection light quantity Iw before color development and detection light quantity Ic after color development, respectively. The present calibration method, however, can understandably obtain the same effect also by executing addition or subtraction on any one of the detection light quantities Iw and Ic. In particular, applying equation (1) is further preferable because it is possible to suppress deterioration of measurement accuracy in a case where the degree of contribution of the surface reflection light 110 and 116 is relatively large, that is, where the detection light quantity ratio is small.

Furthermore, in the above-stated embodiment, the correction light quantity $\Delta I$ is determined as the calibration coefficient 64. The calibration coefficient, however, is not limited to the present embodiment. For example, when the relationship between $\eta$ = G1 (Ic/Iw) and Ab = G2 (1024-Iw/Ic) approximately holds, the calibration coefficient 64 may be a coefficient that specifies any of the functions G1 and G2.

[Effects of Calibration Method]

In this manner, the calibration system 70 calibrates the blood glucose meter 10 configured to include the measurement optical system 40 for projecting light on the measurement surface 54 of the test strip 26 that contains a chromogenic reagent that reacts with a component in a body fluid and detecting reflected light and configured to measure the component in the body fluid based on a detection light quantity of the reflected light obtained by the measurement optical system 40.

The calibration system 70 includes the offset measurement means 98 and the calibration curve determination means (calibration curve determination section 96). The offset measurement means 98 is configured to measure the offset (AX, ΔY) of the reflection point R on the measurement surface 54. The offset (ΔX, ΔY) represents a deviation amount of the actual optical path Lr of the measurement optical system 40 with respect to the reference optical path Ls of the measurement optical system 40. The measurement optical system 40 is mounted on the calibration target instrument 10c that is the blood glucose meter 10 as a calibration target. The calibration curve determination means (calibration curve determination section 96) is configured to calculate the correction light quantity (ΔI) to be uniformly added or subtracted to/from the detection light quantity based on the measured offset and configured to determine the calibration curve (calibration coefficient 64) of the calibration target instrument 10c based on the correction light quantity.

The reflected light to be detected by the measurement optical system 40 includes not only scattered light that has been scattered inside the test strip 26 and emitted to the outside, but also the surface reflection light 110 and 116 reflected as they are on the measurement surface 54 of the test strip 26. The surface reflection light 110 and 116 each has an angle distribution such that light intensity is maximized at a specular reflection angle, and tends to have a uniform light intensity regardless of the amount of component in the body fluid. That is, a changing optical path (namely, reflection angle) of the measurement optical system 40 leads to a different detection light quantity attributed to the surface reflection light 110 and 116.

Accordingly, the above configuration, if adopted, can execute calibration that cancels a change in the detection light quantity attributed to the surface reflection light 110 and 116. Consequently, it is possible to ensure accuracy of measuring a component in a body fluid without precisely managing mounting accuracy of the measurement optical system 40.

The present invention is not limited to the embodiment described herein and may be appropriately modified without departing from the main scope of the invention.

In the description of the above-described embodiment, blood is used as an example of the body fluid. The present invention, however, is not limited to blood. For example, the body fluid may be lymph fluid, spinal fluid, saliva or the like.

Furthermore, the component of the body fluid may be: glucose (blood glucose level) and others such as cholesterol, uric acid, creatinine, lactic acid, hemoglobin (occult blood), various alcohols, various saccharides, various proteins, various vitamins, various inorganic ions including sodium, and endocrine-disrupting chemicals including polychlorinated biphenyl (PCB) and dioxin. Furthermore, it is possible to configure to acquire, as measurement results, quality of the component, separately from or additionally to the quantity of the component.

**Claims**

1. A calibration method for calibrating a body fluid component measuring instrument (10) configured to include a measurement optical system (40) for projecting light on a measurement surface (54) of a test strip (26) that contains a chromogenic reagent that reacts with a component in a body fluid and detecting reflected light, and configured to measure the component in the body fluid based on a detection light quantity of the reflected light obtained by the measurement optical system (40), the calibration method comprising:

   a measurement step of measuring an offset of a reflection point (R) on the measurement surface (54), the offset representing a deviation amount of an actual optical path (Lr) of the measurement optical system (40) with respect to a reference optical path (Ls) of the measurement optical system (40), the measurement optical system (40) being mounted on a calibration target instrument (10c) that is the body fluid component measuring instrument (10) as a calibration target; and
   a determination step of calculating a correction light quantity to be uniformly added or subtracted to/from the detection light quantity based on the measured offset and determining a calibration curve for the calibration target instrument (10c) based on the correction light quantity.

2. The calibration method according to claim 1, wherein the measurement step measures, as the offset, deviation of the reflection point (R) along an intersection line formed by a surface that contains the reference optical path (Ls) and the measurement surface (54).

3. The calibration method according to claim 1, wherein the body fluid component measuring instrument (10) measures the component in the body fluid based on a ratio of the detection light quantities on the test strip (26) between before and after color development, and the determination step determines the calibration curve based on a correction model configured to add or subtract the correction light quantity to/from each of the detection light quantities, respectively, before and after color development.

4. The calibration method according to claim 1, wherein the determination step acquires association information (90) that represents a relationship of the correction light quantity with respect to the offset and then calculates the correction light quantity with reference to the association information (90).

5. A calibration system (70) for calibrating a body fluid component measuring instrument (10) configured to include a measurement optical system (40) for projecting light on a measurement surface (54) of a test strip (26) that contains a chromogenic reagent that reacts with a component in a body fluid and for detecting reflected light, and configured to measure the component in the body fluid based on a detection light quantity of the reflected light obtained by the measurement optical system (40), the calibration system comprising:

offset measurement means (98) configured to measure an offset of a reflection point (R) on the measurement surface (54), the offset representing a deviation amount of an actual optical path (Lr) of the measurement optical system (40) with respect to a reference optical path (Ls) of the measurement optical system (40), the measurement optical system (40) being mounted on a calibration target instrument (10c) that is the body fluid component measuring instrument (10) as a calibration target; and

calibration curve determination means (96) configured to calculate a correction light quantity to be uniformly added or subtracted to/from the detection light quantity based on the offset measured by the offset measurement means (98) and configured to determine a calibration curve for the calibration target instrument (10c) based on the correction light quantity.

6. A body fluid component measuring instrument (10) configured to include a measurement optical system(40) for projecting light on a measurement surface (54) of a test strip (26)that contains a chromogenic reagent that reacts with a component in a body fluid and for detecting reflected light, and configured to measure the component in the body fluid based on a detection light quantity of the reflected light obtained by measurement optical system (40), said body fluid component measuring instrument (10) having been calibrated by using the calibration method according to any one of claims 1 to 4, comprising:

a storage unit (44) configured to store a calibration coefficient (64) that specifies the determined calibration curve; and

a component quantification section (60) configured to use the calibration coefficient (64) read from the storage unit (44) to uniformly add or subtract the correction light quantity to/from the detection light quantity obtained by the measurement optical system (40) and then to quantify the component in the body fluid.

**Patentansprüche**

1. Kalibrierungsverfahren zum Kalibrieren eines Messinstruments (10) für Körperflüssigkeitskomponenten, das so konfiguriert ist, dass es ein optisches Messsystem (40) zum Projizieren von Licht auf eine Messoberfläche (54) eines Teststreifens (26), der ein chromogenes Reagenz enthält, das mit einer Komponente in einer Körperflüssigkeit reagiert, und zum Erfassen von reflektiertem Licht umfasst und das so konfiguriert ist, dass es die Komponente in der Körperflüssigkeit auf der Grundlage einer Erfassungslichtmenge des reflektierten Lichts, die durch das optische Messsystem (40) erhalten wird, misst, wobei das Kalibrierungsverfahren umfasst:

einen Messschritt des Messens eines Versatzes eines Reflexionspunktes (R) auf der Messoberfläche (54), wobei der Versatz einen Abweichungsbetrag eines tatsächlichen optischen Pfades (Lr) des optischen Messsystems (40) in Bezug auf einen optischen Referenzpfad (Ls) des optischen Messsystems (40) darstellt, wobei das optische Messsystem (40) auf einem Kalibrierungsziel-Instrument (10c), welches das Messinstrument (10) für Körperflüssigkeitskomponenten ist, als ein Kalibrierungsziel angebracht ist; und

einen Bestimmungsschritt des Berechnens einer Korrekturlichtmenge, die, auf der Grundlage des gemessenen Versatzes, einheitlich zu der Erfassungslichtmenge zu addieren oder von ihr zu subtrahieren ist, und des Bestimmens einer Kalibrierungskurve für das Kalibrierungsziel-Instrument (10c) auf der Grundlage der Korrekturlichtmenge.

2. Kalibrierungsverfahren nach Anspruch 1, wobei der Messschritt als den Versatz die Abweichung des Reflexionspunktes (R) entlang einer Schnittlinie misst, welche durch eine Fläche, die den optischen Referenzpfad (Ls) enthält, und die Messfläche (54) gebildet wird.

3. Kalibrierungsverfahren nach Anspruch 1, wobei das Messinstrument (10) für Körperflüssigkeitskomponenten die Komponente in der Körperflüssigkeit auf der Grundlage eines Verhältnisses zwischen den Erfassungslichtmengen auf dem Teststreifen (26) vor und nach der Farbentwicklung misst und der Bestimmungsschritt die Kalibrierungskurve auf der Grundlage eines Korrekturmodells bestimmt, das so konfiguriert ist, dass es die Korrekturlichtmenge entsprechend zu jeder der Erfassungslichtmengen vor und nach der Farbentwicklung addiert oder davon subtrahiert.

4. Kalibrierungsverfahren nach Anspruch 1, wobei der Bestimmungsschritt Assoziationsinformationen (90) erlangt, die eine Beziehung der Korrekturlichtmenge in Bezug auf den Versatz darstellen, und anschließend die Korrekturlichtmenge unter Bezugnahme auf die Assoziationsinformationen (90) berechnet.

5. Kalibrierungssystem (70) zum Kalibrieren eines Messinstruments (10) für Körperflüssigkeitskomponenten, das so konfiguriert ist, dass es ein optisches Messsystem (40) zum Projizieren von Licht auf eine Messoberfläche (54) eines Teststreifens (26), der ein chromogenes Reagenz enthält, das mit einer Komponente in einer Körperflüssigkeit reagiert, und zum Erfassen von reflektiertem Licht umfasst, und das so konfiguriert ist, dass es die Komponente in der Körperflüssigkeit auf der Grundlage einer Erfassungslichtmenge des reflektierten Lichts, die durch das optische Messsystem (40) erhalten wird, misst, wobei das Kalibrierungsverfahren umfasst:

eine Versatz-Messeinrichtung (98), die zum Messen eines Versatzes eines Reflexionspunktes (R) auf der Messoberfläche (54) konfiguriert ist, wobei der Versatz einen Abweichungsbetrag eines tatsächlichen optischen Pfades (Lr) des optischen Messsystems (40) in Bezug auf einen optischen Referenzpfad (Ls) des optischen Messsystems (40) darstellt, wobei das optische Messsystem (40) auf einem Kalibrierungsziel-Instrument (10c), welches das Messinstrument (10) für Körperflüssigkeitskomponenten ist, als ein Kalibrierungsziel angebracht ist; und
eine Kalibrierungskurven-Bestimmungseinrichtung (96), die zum Berechnen einer Korrekturlichtmenge konfiguriert ist, die, auf der Grundlage von dem durch die Versatz-Messeinrichtung (98) gemessenen Versatz, einheitlich zu der Erfassungslichtmenge zu addieren oder von ihr zu subtrahieren ist und die zum Bestimmen einer Kalibrierungskurve für das Kalibrierungsziel-Instrument (10c) auf der Grundlage der Korrekturlichtmenge konfiguriert ist.

6. Messinstrument (10) für Körperflüssigkeitskomponenten, das so konfiguriert ist, dass es ein optisches Messsystem (40) zum Projizieren von Licht auf eine Messoberfläche (54) eines Teststreifens (26), der ein chromogenes Reagenz enthält, das mit einer Komponente in einer Körperflüssigkeit reagiert, und zum Erfassen von reflektiertem Licht umfasst, und das so konfiguriert ist, dass es die Komponente in der Körperflüssigkeit auf der Grundlage einer Erfassungslichtmenge des reflektierten Lichts, die durch das optische Messsystem (40) erhalten wird, misst, wobei das Messinstrument (10) für Körperflüssigkeitskomponenten, das durch Verwendung des Kalibrierungsverfahrens nach einem der Ansprüche 1 bis 4 kalibriert wurde, umfasst:

eine Speichereinheit (44), die so konfiguriert ist, dass sie einen Kalibrierungskoeffizienten (64) speichert, der die bestimmte Kalibrierungskurve spezifiziert; und
einen Komponentenquantifizierungsabschnitt (60), der so konfiguriert ist, dass er den aus der Speichereinheit (44) ausgelesenen Kalibrierungskoeffizienten (64) verwendet, um die Korrekturlichtmenge einheitlich zu der durch das optische Messsystem (40) erhaltenen Erfassungslichtmenge zu addieren oder von ihr zu subtrahieren und anschließend die Komponente in der Körperflüssigkeit zu quantifizieren.

## Revendications

1. Procédé d'étalonnage pour étalonner un instrument de mesure de composant de fluide corporel (10) configuré pour inclure un système optique de mesure (40) pour projeter de la lumière sur une surface de mesure (54) d'une bandelette de test (26) qui contient un réactif chromogénique qui réagit avec un composant dans un fluide corporel et détecter la lumière réfléchie, et configuré pour mesurer le composant dans le fluide corporel sur la base d'une quantité de lumière de détection de la lumière réfléchie obtenue par le système optique de mesure (40), le procédé d'étalonnage comprenant :

une étape de mesure pour mesurer un décalage d'un point de réflexion (R) sur la surface de mesure (54), le décalage représentant une quantité de déviation d'un trajet optique réel (Lr) du système optique de mesure (40) par rapport à un trajet optique de référence (Ls) du système optique de mesure (40), le système optique de mesure (40) étant monté sur un instrument cible d'étalonnage (10c) qui est l'instrument de mesure de composant de fluide corporel (10) en tant que cible d'étalonnage ; et

une étape de détermination pour calculer une quantité de lumière de correction à ajouter ou soustraire uniformément à/de la quantité de lumière de détection sur la base du décalage mesuré et déterminer une courbe d'étalonnage pour l'instrument cible d'étalonnage (10c) sur la base de la quantité de lumière de correction.

2. Procédé d'étalonnage selon la revendication 1, dans lequel l'étape de mesure mesure, en tant que décalage, une déviation du point de réflexion (R) le long d'une ligne d'intersection formée par une surface qui contient le trajet optique de référence (Ls) et la surface de mesure (54).

3. Procédé d'étalonnage selon la revendication 1, dans lequel l'instrument de mesure de composant de fluide corporel (10) mesure le composant dans le fluide corporel sur la base d'un rapport des quantités de lumière de détection sur la bandelette de test (26) entre avant et après le développement des couleurs, et l'étape de détermination détermine la courbe d'étalonnage sur la base d'un modèle de correction configuré pour ajouter ou soustraire la quantité de lumière de correction à/de chacune des quantités de lumière de détection, respectivement, avant et après le développement des couleurs.

4. Procédé d'étalonnage selon la revendication 1, dans lequel l'étape de détermination acquiert des informations d'association (90) qui représentent une relation de la quantité de lumière de correction par rapport au décalage et calcule ensuite la quantité de lumière de correction en se référant aux informations d'association (90).

5. Système d'étalonnage (70) pour étalonner un instrument de mesure de composant de fluide corporel (10) configuré pour inclure un système optique de mesure (40) pour projeter de la lumière sur une surface de mesure (54) d'une bandelette de test (26) qui contient un réactif chromogénique qui réagit avec un composant dans un fluide corporel et pour détecter la lumière réfléchie, et configuré pour mesurer le composant dans le fluide corporel sur la base d'une quantité de lumière de détection de la lumière réfléchie obtenue par le système optique de mesure (40), le système d'étalonnage comprenant :

un moyen de mesure de décalage (98) configuré pour mesurer un décalage d'un point de réflexion (R) sur la surface de mesure (54), le décalage représentant une quantité de déviation d'un trajet optique réel (Lr) du système optique de mesure (40) par rapport à un trajet optique de référence (Ls) du système optique de mesure (40), le système optique de mesure (40) étant monté sur un instrument cible d'étalonnage (10c) qui est l'instrument de mesure de composant de fluide corporel (10) en tant que cible d'étalonnage ; et

un moyen de détermination de courbe d'étalonnage (96) configuré pour calculer une quantité de lumière de correction à ajouter ou soustraire uniformément à/de la quantité de lumière de détection sur la base du décalage mesuré par le moyen de mesure de décalage (98) et configuré pour déterminer une courbe d'étalonnage pour l'instrument cible d'étalonnage (10c) sur la base de la quantité de lumière de correction.

6. Instrument de mesure de composant de fluide corporel (10) configuré pour inclure un système optique de mesure (40) pour projeter de la lumière sur une surface de mesure (54) d'une bandelette de test (26) qui contient un réactif chromogénique qui réagit avec un composant dans un fluide corporel et pour détecter la lumière réfléchie, et configuré pour mesurer le composant dans le fluide corporel sur la base d'une quantité de lumière de détection de la lumière réfléchie obtenue par le système optique de mesure (40), ledit instrument de mesure de composant de fluide corporel (10) ayant été étalonné en utilisant le procédé d'étalonnage selon l'une quelconque des revendications 1 à 4, comprenant :

une unité de stockage (44) configurée pour stocker un coefficient d'étalonnage (64) qui spécifie la courbe d'étalonnage déterminée ; et

une section de quantification de composant (60) configurée pour utiliser le coefficient d'étalonnage (64) lu à partir de l'unité de stockage (44) pour ajouter ou soustraire uniformément la quantité de lumière de correction à/de la quantité de lumière de détection obtenue par le système optique de mesure (40) et ensuite pour quantifier le composant dans le fluide corporel.

EP 2 950 082 B1

*FIG. 1*

FIG. 2

FIG. 3

EP 2 950 082 B1

15

## FIG. 4

START

ACQUIRE REFERENCE POSITION — S1

PREPARE CALIBRATION TARGET INSTRUMENT — S2

IMAGE PERIPHERY OF MEASUREMENT OPTICAL SYSTEM — S3

CALCULATE OFFSET — S4

DETERMINE CALIBRATION CURVE — S5

SET AND INPUT CALIBRATION COEFFICIENT — S6

CALIBRATION COMPLETED ON ALL INSTRUMENTS? — S7 NO

YES

END

# FIG. 5

# FIG. 6

EP 2 950 082 B1

## FIG. 7A

## FIG. 7B

FIG. 8

EP 2 950 082 B1

## FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012147504 A
- WO 2012128014 A

- JP 9145615 A